# FASCICULE DE BREVET EUROPEEN

(11) **EP 3 427 665 B1**
(45) Date de publication et mention de la délivrance du brevet: **09.10.2024**
(21) Numéro de dépôt: 18182657.9
(22) Date de dépôt: 10.07.2018
(51) Int. Cl.: A61B 6/00, A61B 6/58

(54) **PROCEDE ET SYSTEME DE CALIBRATION EN LIGNE D'UN DISPOSITIF MEDICAL A RAYONS X**
VERFAHREN UND SYSTEM ZUR ONLINE-KALIBRIERUNG VON MEDIZINISCHEN RÖNTGENGERÄTEN
METHOD AND SYSTEM FOR ONLINE CALIBRATION OF A MEDICAL X-RAY DEVICE

(30) Priorité: 11.07.2017 FR 1700740
(43) Date de publication de la demande: 16.01.2019
(73) Titulaire: THALES, 92400 Courbevoie (FR)
(72) Inventeur: GORGES, Sébastien, 38430 Saint Jean de Moirans (FR); BERNARD, Guillaume, 38430 Moirans (FR)
(74) Mandataire: Atout PI Laplace

(56) Documents cités:
- US-A1- 2008 130 827
- US-A1- 2012 236 999
- US-A1- 2014 010 349
- US-A1- 2016 278 732

## Description

L'invention concerne notamment un procédé et un système de calibration en ligne d'un dispositif médical à rayons X et plus particulièrement les systèmes de radiologie utilisés pour la chirurgie ou la radiologie interventionnelle comme les arceaux mobiles chirurgicaux. Elle est notamment utilisée pour calibrer en ligne des appareils dits en arceau.

Il est courant d'utiliser un système de radiologie interventionnel mobile pour effectuer des procédures chirurgicales ou interventionnelles. Ces systèmes, aussi appelés mobiles C-arm (arceaux chirurgicaux ou amplificateur de bloc) permettent au chirurgien d'acquérir des images rayons X durant l'intervention et de contrôler en temps réel le positionnement des outils (cathéter, aiguille, prothèse, etc.) de manière minimalement invasive. La plupart de ces systèmes permettent d'obtenir des images en deux dimensions avec un flux d'image vidéo jusqu'à trente images par seconde. Ces systèmes comprennent généralement une source de rayons X et un détecteur d'images monté aux deux extrémités d'un bras en C, entre lesquelles est positionné un objet dont on souhaite réaliser l'image. Les images acquises par le détecteur sont le résultat d'une projection de l'objet dans le plan du détecteur.

Le praticien utilise ensuite ces images en deux dimensions 2D pour effectuer, en temps réel, une reconstruction mentale de la morphologie du patient, lui permettant de positionner en temps réel l'instrument utilisé par rapport à une zone à opérer. Il effectue également et mentalement la réorientation dans l'espace de la scène complète (patient + instruments chirurgicaux) afin de contrôler de manière précise son geste.

Plus récemment, des systèmes sophistiqués ont fait leur apparition. Ils permettent d'acquérir une image 3D de l'instrument chirurgical pendant l'intervention. Le système effectue une rotation autour du patient dans le but d'obtenir un ensemble d'images en 2D. Ces images 2D sont ensuite traitées par un algorithme de reconstruction d'images permettant l'obtention d'une image volumique en 3D. L'algorithme de reconstruction a besoin de connaître la géométrie exacte du C-arm, à savoir, la position du détecteur et du tube à rayons X par rapport au patient ou à un objet, pour chaque image 2D exploitée. Les systèmes actuels proposent d'effectuer une calibration « hors ligne », en utilisant par exemple une mire de calibration 3D pour déterminer les matrices de projection. Cette calibration « hors ligne » est effectuée pendant les phases de maintenance préventive que subit le système, par exemple tous les six mois ou tous les ans.

La demande de brevet EP3141187 concerne une mire de calibration pour calibrer géométriquement un dispositif d'imagerie par rayons X destiné à générer des images tridimensionnelles d'un objet par reconstruction à partir de projections bidimensionnelles dudit objet. La mire de calibration comprend un support volumique muni de marqueurs à absorption radiologique contrastée par rapport au support volumique, les marqueurs étant répartis selon une figure tridimensionnelle. Les marqueurs sont répartis en sous-ensembles de marqueurs distribués selon des droites respectives sensiblement parallèles de façon que des séquences de birapports puissent être construites à partir des sous-ensembles de marqueurs respectifs. Chaque séquence de birapports comprend un unique birapport pour chaque quadruplet de marqueurs dans lequel les marqueurs sont ordonnés selon un ordre dépendant des numéros d'ordre des marqueurs respectifs le long de la droite selon laquelle ils sont alignés, selon un premier sens prédéfini, ledit ordre étant commun à tous les birapports.

Une technique de calibration connue de l'art antérieur utilise par exemple des marqueurs positionnés sur un fantôme qui servent de repères dans l'espace. La position des marqueurs dans l'espace étant connue, il est possible de déduire la géométrie de l'acquisition pour chaque projection par inversion d'un système d'équations dérivé de la position des marqueurs sur les images projetées.

Les systèmes connus de l'art antérieur supposent que l'acquisition rotationnelle est suffisamment répétable pour que la géométrie déterminée « hors ligne » soit applicable sur les images acquises en temps réel pendant l'intervention. La mécanique des systèmes a donc dû être améliorée afin de rendre stable l'arceau pendant l'acquisition rotationnelle des images 2D (réduction de jeux mécaniques, pièces plus rigides, ..). Ces systèmes sont peu répandus car leur coût est élevé du fait des modifications mécaniques devant être apportées. De plus, il n'est pas trivial pour les fabricants du dispositif de type C-arm d'implémenter ces modifications.

Certaines solutions connues de l'art antérieur proposent d'effectuer « en ligne » une calibration avec une méthode basée sur des capteurs directement intégrés sur le dispositif et sans faire appel à de l'analyse d'images contenant des marqueurs radio-opaques.

Une première solution utilise un simple capteur inertiel trois axes positionné sur le détecteur ou sur la source de rayons. Une telle méthode est décrite dans le document de Grzeda Victor et al, intitulé «C-arm rotation encoding with accelerometer », International Journal of Computer Assisted Radiology and Surgery, 2010, 5(4), pp : 385-391.

Une seconde solution utilise deux centrales à inertie six axes et deux télémètres laser, comme par exemple, dans le document de Amiri Shahram, Wilson David R., intitulé « A low-cost tracked C-arm (TC-arm) upgrade system for versatile quantitative intraoperative imaging », International Journal of Computer Assisted Radiology and Surgery, 9(4), pp :695-711, 2014.

Dans le cas d'utilisation demandant une très grande précision, par exemple dans le domaine de la chirurgie, ces méthodes ne sont pas optimales. En effet, la précision atteinte n'est pas compatible avec la qualité de reconstruction 3D souhaitée.

Dans le document de Grzeda Victor, l'idée de localiser le détecteur et la source de Rayons X à l'aide d'un accéléromètre à trois axes en adoptant les principes introduits par la navigation a été abandonnée au profit de l'idée d'estimer l'angle de rotation au niveau de son axe de rotation (remplacement d'un codeur rotatif à l'aide d'un accéléromètre) et de déduire, en ligne, les positions d'un système à trajectoire reproductible calibré au préalable.

Avec un dispositif comprenant deux centrales à inertie six axes et deux télémètres laser, document précité de Amiri Sharham, les précisions obtenues sont de l'ordre de :
- 1,5 mm +/- 1,2 mm de précision pour la localisation de l'isocentre du système,
- 2,3 mm +/- 1,1 mm de précision pour le recalage 2D-3D,
- 4,4 mm +/- 1,9 mm de précision de localisation de marqueurs dans la scène reconstruite.

Ces valeurs de précisions sont nettement en dessous des valeurs de précision requises pour des applications précises et minutieuses, notamment dans des applications chirurgicales, ou dans d'autres domaines requérant de la précision, par exemple en métrologie de pièces en fin de fabrication.

La demande de brevet US 2016/278732 concerne un système qui inclut un système de suivi de position de la source et de la position du détecteur par mise en correspondance des mesures du mouvement des articulations du dispositif (articulations ou liaisons de type axe de rotation et bras de translation) avec les paramètres de calibration mémorisés a priori pour toutes les positions possibles. De plus, dans ce document les capteurs sont décrits comme des encodeurs montés sur ces articulations pour mesurer le mouvement relatif de ces liaisons uniquement et non sur le capteur et la source.

La demande de brevet US 2012/236999 décrit un dispositif C-arm équipé d'une jauge de contrainte qui renseigne de la déformation du C-arm d'une position à une autre. Fort de cette information, le contrôleur du système compense cette déformation en adaptant la consigne des actionneurs de position de façon à corriger ladite déformation.

L'idée de la présente invention concerne une méthode et un système de calibration permettant d'estimer la géométrie en ligne d'un dispositif, en cours de fonctionnement. La calibration se fera pendant l'acquisition rotationnelle du dispositif afin de ne pas contraindre les procédures de maniement du dispositif et, en particulier, de ne pas requérir à des calibrations a priori (hors ligne) lourdes et contraignantes. L'invention trouve notamment son application dans le domaine des opérations chirurgicales, de manière plus large à une utilisation clinique, non-contraignante et avec un temps d'exécution rapide.

Dans la description, les termes « appareil » et « dispositif » désignent un même objet.

L'expression « géométrie » de système désigne l'ensemble des neuf paramètres intrinsèques (caractérisation du couple source RX/détecteur) et extrinsèques (caractérisation de la géométrie du couple patient//source RX/détecteur).

L'invention concerne un système de calibration d'un dispositif D défini selon les revendications indépendantes. D'autres modes de réalisation sont définis dans les revendications dépendantes.

D'autres caractéristiques et avantages de la présente invention apparaîtront mieux à la lecture de la description d'exemples de réalisation annexée des figures qui représentent :
- Figure 1, une représentation schématique d'un appareil d'imagerie du type à bras en C, ou C-arm,
- Figure 2, un schéma d'un module de traitement des données, et
- Figure 3, un synoptique des étapes mises en oeuvre lors de la calibration selon l'invention.

Afin de mieux faire comprendre l'objet de la présente invention, l'exemple qui suit est donné à titre illustratif pour un dispositif de type C-arm utilisé dans le domaine chirurgical. Sans sortir du cadre de l'invention, l'invention pourrait être mise en oeuvre dans tout dispositif comprenant au moins une source de rayonnement et un détecteur, la source et le détecteur étant montés sur un ou plusieurs supports en mouvement, le dispositif étant calibré en ligne (en cours du fonctionnement du dispositif) pour optimiser les résultats de mesure.

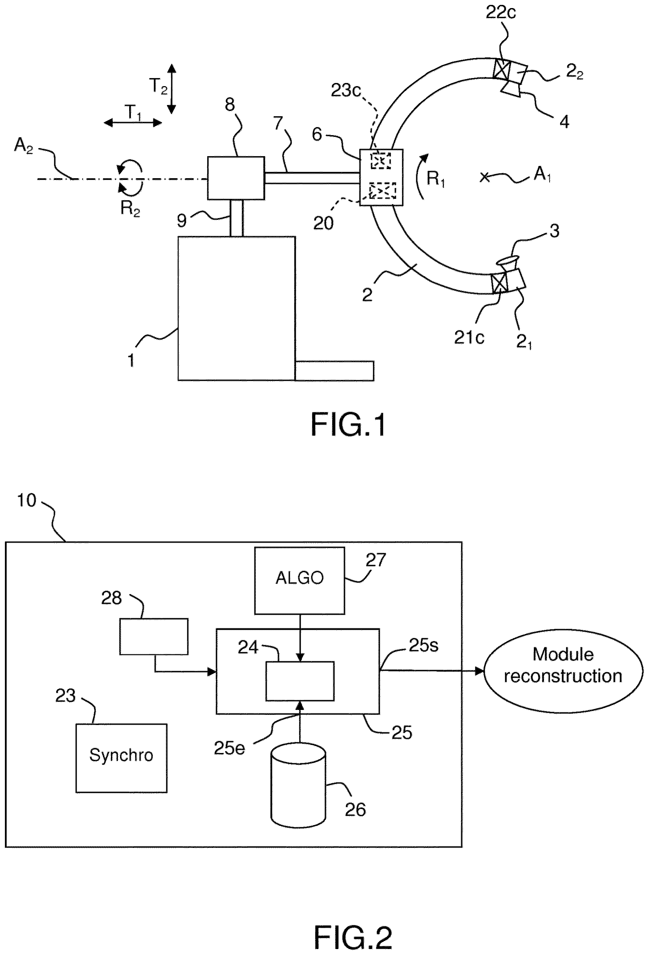

Sur la figure 1, le dispositif D représenté comprend une base 1 sur laquelle est montée un bras en forme de « C », 2. Le bras en « C » supporte à une première extrémité 2₁ une source de rayonnement 3 constituée par exemple d'un tube à rayons X et à une deuxième extrémité 2₂ un détecteur de rayons X, 4, qui fournit une image 2D. Le bras en « C » est positionné dans une glissière en forme d'arc creux 6 dans lequel il glisse selon un mouvement de rotation « orbitale » R1, dont l'axe A1 est le centre d'un cercle représenté en partie par le bras en arceau. La glissière est fixée à la base 1 via une pièce de maintien 8 et d'un bras 7 permettant d'assurer un deuxième mouvement de rotation « angulaire » R2 selon un deuxième axe A2.

Pour la calibration en ligne de l'appareil, le dispositif comprend un premier capteur tel qu'un capteur inertiel 21c disposé à proximité de la source 3 et au moins un deuxième capteur tel qu'un capteur inertiel 22c disposé à proximité du détecteur 4. Les deux capteurs inertiels 21c, 22c sont adaptés à effectuer des mesures d'accélération et de vitesse qui permettront d'en déduire une position Ps de la source et une position Pd du détecteur selon des principes connus de l'homme du métier.

Le capteur adapté à effectuer des mesures d'accélération et de vitesse peut être, un capteur inertiel, un capteur ultrason, un capteur optique, un encodeur ou tout autre dispositif connu de l'homme du métier et qui assure la fonction de mesure à partir de laquelle on peut calculer ou estimer des positions.

Le dispositif peut aussi comporter un capteur de type encodeur 20 adapté à mesurer la position angulaire Pa du mouvement orbital du bras en C à un instant t donné. L'utilisation d'un encodeur permet une mesure plus simple et plus fiable. Certains types de capteurs seront donnés à titre d'exemples plus loin dans la description.

Afin d'effectuer les mesures, le dispositif est relié à un module de pilotage et de traitement des données 10 (figure 2) comprenant les éléments suivants : un module de synchronisation 23 permettant de déclencher des mesures de manière synchrone au niveau des capteurs équipant le bras, un processeur 24 exécutant les étapes du procédé selon l'invention, un module de prétraitement 25 des mesures effectuées par les capteurs.

Le module de synchronisation 23 permet d'horodater simultanément les acquisitions d'images et les mesures des capteurs inertiels et de l'encodeur équipant le bras. Les résultats de mesure (capteurs inertiels et bras) se présentent par exemple sous la forme d'un tableau ou d'une base de données contenant pour un instant ti donné, une acquisition d'images 2D et trois mesures.

Selon la figure 2, le module de prétraitement comprend une entrée 25e recevant un modèle de fonctionnement dynamique 26 de l'appareil et un algorithme de fusion de données 27, de type Kalman par exemple ou de type Kalman étendu, prenant en compte au moins les trois mesures, des deux capteurs inertiels et de l'encodeur, 28, et le modèle de fonctionnement dynamique de l'appareil 26 pour en déduire une matrice de projection qui sera transmise via une sortie 25s du module de prétraitement à un module de reconstruction.

Le nombre d'encodeurs utilisés est choisi, par exemple, en fonction du nombre de degrés de liberté (rotation-translation) de l'appareil. Les encodeurs ont notamment pour fonction de déterminer avec une faible précision, mais de manière stable, la position absolue de l'appareil par rapport à un référentiel lié à la base de l'appareil (coordonnées de l'appareil du C-arm sur lequel sont fixés les roulettes ou point de référence par exemple), et ainsi de connaître une première approximation des paramètres intrinsèques et extrinsèques ou encore la matrice de projection 4×3 3D/2D associée qui sera utilisée selon des techniques connues d'un homme du métier pour reconstruire une image en 3D.

Pour déterminer la position du bras « C » on utilisera, par exemple, une règle linéaire potentiométrique de positionnement pour le mouvement « orbital » R1, un encodeur à roue codeuse optique pour le mouvement « angulaire » R2 et deux mouvements rectilignes pour les mouvements T1 (« avant/arrière ») et T2 (« haut/bas »), figure 1.

Pour le déplacement du bras, ce dernier peut être équipé d'une courroie souple à dents et la glissière 6, support du bras, d'une roue crantée qui entraîne la courroie souple.

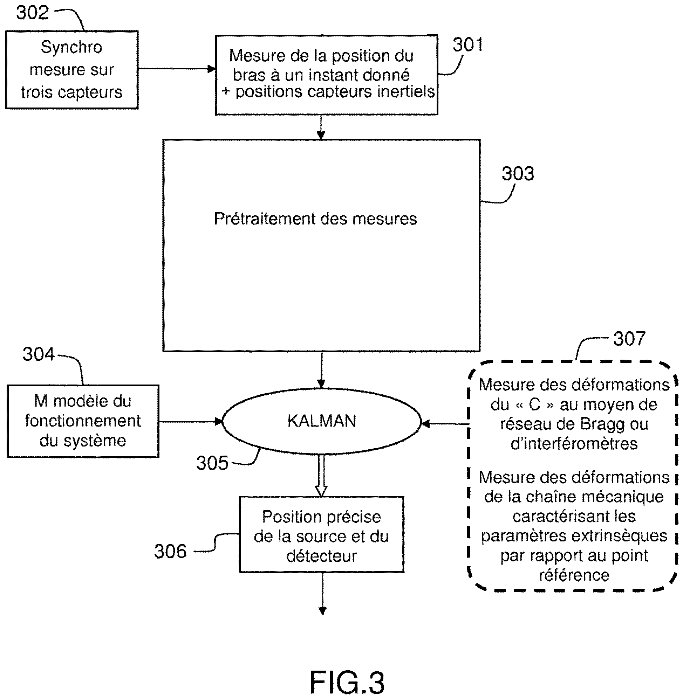

La figure 3 représente un organigramme listant l'enchaînement des étapes mises en oeuvre par le procédé.

Dans l'exemple qui suit, le procédé utilise par exemple l'encodeur 20 pour relever la position du bras en « C » et au moins les deux capteurs inertiels positionnés au niveau de chacune des extrémités du bras, où se trouvent la source et le détecteur. Les mesures sont effectuées 301 de manière synchrone 302 grâce au module de synchronisation qui transmet un ordre de déclenchement des mesures simultanément vers l'encodeur et les deux capteurs inertiels. Les mesures des capteurs inertiels (accélération et vitesse angulaire) sont enregistrées et prétraitées. Le prétraitement consiste par exemple à filtrer ces mesures, à extraire une valeur de biais et à rééchantillonner les mesures. Le processeur va aussi établir un modèle dynamique M du fonctionnement du dispositif D, selon des principes connus de l'homme du métier ou bien un modèle de fonctionnement dynamique sera stocké dans une base de données et envoyé vers le processeur.

Les deux mesures effectuées par les deux capteurs inertiels 21c, 22c, et la mesure Pa réalisée par l'encodeur, sont transmises 303 au module de prétraitement des données, 25. Dans cet exemple, un filtre de Kalman étendu est utilisé. Il est aussi possible d'utiliser un filtre ayant une fonctionnalité équivalente. Le filtre de Kalman étendu, 305, reçoit en entrée le modèle dynamique M du fonctionnement de l'appareil, 304, et les trois mesures Ps, Pd et Pa issues du module de traitement des données. L'étape de fusion des données réalisée par le filtre de Kalman étendu permet notamment d'estimer la pose (position et orientation) du détecteur et de la source ainsi que l'erreur réalisée sur cette estimation. A partir de la fusion de ces données, le filtre de Kalman étendu estime une position de la source de rayons X et une position précise du détecteur, 306. Les précisions des positions obtenues seront par exemple comprises dans l'intervalle [100 µm-500 µm]. La matrice de projection 3D/2D sera ensuite calculée à partir des positions précises.

La précision résulte notamment du fait que le système selon l'invention est non causal, l'estimation ne se faisant pas en temps réel, ce qui permet d'utiliser des filtres arrière-avant ou « Backward-Forward ».

Un modèle M de fonctionnement dynamique du dispositif peut être construit. Malgré la non-reproductibilité du mouvement du C-arm, l'écart entre deux courses reste limité. Une trajectoire moyenne de l'arceau peut être établie et intégrée au modèle. Des modèles précis des capteurs utilisés (modèle inertiel) sont établis et intégrés dans les modèles d'observation du filtre de Kalman étendu.

Il est aussi possible d'utiliser des capteurs hétérogènes (capteurs inertiels ou de même nature) afin de profiter de la redondance de l'information de pose et permettre un meilleur lissage des erreurs non corrélées.

Afin d'augmenter la précision dans la position obtenue, le dispositif peut être équipé d'un réseau de capteurs inertiels de type MEMs à six ou neuf degrés de liberté placés à des positions choisies. Du fait de leur faible coût et de leur simplicité de mise en oeuvre, le nombre de capteurs inertiels peut être important.

Par exemple, une solution consiste à positionner des capteurs inertiels de la manière suivante :
- Un premier capteur inertiel de référence lié au référentiel système,
- Un capteur inertiel sur le point de rotation du bras « C » (au niveau du couplage mécanique pour faire le mouvement orbital),
- Un capteur inertiel sur le point milieu du bras « C », ou répartis sur tout le long du bras,
- Trois ou quatre capteurs inertiels au niveau du détecteur et de la source Rx fixés aux deux extrémités du bras « C ».
Le capteur 22c est, par exemple, un capteur inertiel à six degrés de liberté minimum : trois accéléromètres et trois gyromètres, le capteur 21c est un capteur inertiel à trois degrés de liberté minimum : trois accéléromètres.

Sur la figure 1, un troisième capteur inertiel 23c est positionné à mi-chemin entre les deux capteurs inertiels 21c, 22c.

Selon une variante de réalisation, comme il est décrit ci-après, le filtre de Kalman peut aussi recevoir des mesures complémentaires, (figure 3, 307), telles que les mesures des déformations du bras en « C », obtenues en utilisant un ou plusieurs réseaux de Bragg non représentés pour des raisons de simplification. Ces réseaux peuvent être positionnés le long du bras en « C » sur les faces ou les arêtes pour mesurer la déformation du bras.

Une autre solution permettant d'augmenter la précision consiste à utiliser un ou plusieurs interféromètres de Mach Zehnder ou de Michelson. Ils permettront de mesurer via une ou plusieurs tiges souples, par exemple non extensibles, non compressibles et placées dans un gaine aménagée le long du bras en « C ».

En fusionnant l'ensemble des données : données de mesures obtenues par les capteurs inertiels, les encodeurs, les réseaux de Bragg, les interféromètres, l'ensemble des paramètres peut être obtenu avec une meilleure précision. Les mesures des encodeurs permettent notamment de renforcer les mesures des capteurs inertiels.

Afin de réduire le nombre de capteurs électroniques de mesure de la déformation dans toutes les directions de l'espace, une variante prévoit de fusionner les données de tous les capteurs avec les résultats de la modélisation des déformations mécaniques du « C ». En effet, en fonction de la conception du « C », il se peut qu'il soit suffisant de ne mesurer qu'un nombre réduit de déformations tout en conservant une précision suffisante de l'estimation.

Selon une variante de réalisation, l'association de un ou plusieurs télémètres laser permettront de mesurer avec une grande précision, par exemple de l'ordre du micron, les écarts dans les trois dimensions du déplacement du bras « C » par rapport au mécanisme de rotation du bras « C » sur le mouvement orbital par rapport à sa trajectoire théorique. Une variante est d'utiliser un système à balayage laser qui mesure en temps réel une partie du profil du bras en « C ».

Une autre solution se substituant aux solutions de mesure optiques (interféromètre, télémètre lasers...) est d'utiliser une combinaison de capteur RFID combiné à des capteurs ultra son pour mesurer les mêmes écarts ; ces solutions basées sur les mesures relatives de « temps de vols » sont moins précises que les mesures optiques mais aussi moins coûteuses et donc mieux adaptées au système final ciblé.

Une opération de triangulation à partir des mesures permettra ensuite de mesurer les déplacements de la partie mécanique maintenant le bras « C » et autorisant sa rotation orbitale par rapport au référentiel lié au point de référence de l'appareil. Ce mouvement cumule les déplacements « avant/arrière » et « haut/bas ». Tout autre système de suivi de précision pourra être utilisé pour cette mesure complémentaire, par exemple, un système optique, un système à balayage laser 2D ou 3D, etc.

Les paramètres extrinsèques, tels qu'ils ont été définis sont relatifs à un référentiel lié au châssis de l'appareil et non à la table sur laquelle se trouve le patient. La transformation entre les deux référentiels pourra se faire sans difficulté par un homme du métier.

L'invention permet de calibrer le dispositif en ligne, en temps réel, de façon à disposer à tout instant de la position de la source et du détecteur de manière précise. Elle offre la possibilité de déterminer en ligne les paramètres géométriques intrinsèques et extrinsèques d'un C-arm afin d'en déduire de manière précise la matrice de projection 3D/2D qui permet de réaliser des constructions tomographiques 3D de qualité d'un patient mais également de positionner de manière précise le C-arm en cours d'opération chirurgicale.

## Revendications

1. - Système de calibration d'un dispositif médical à rayons X (D) comprenant au moins un support en mouvement (2), une source de rayonnement (3) et un détecteur (4), ledit support en mouvement (2) comprenant deux extrémités (2₁, 2₂), la source de rayonnement (3) et le détecteur (4) étant montés sur ledit au moins un support en mouvement (2) à chaque extrémité (2₁, 2₂), le support en mouvement (2) étant lié à une base (1) via au moins un axe de rotation **caractérisé en ce qu'**il comporte au moins les éléments suivants :
• Au moins un premier capteur (21c) disposé à proximité de la source de rayonnement (3) et au moins un deuxième capteur (22c) disposé à proximité du détecteur (4), les deux premier et deuxième capteurs étant adaptés à estimer par le calcul une position Ps de la source et une position Pd du détecteur en fusionnant des mesures dudit premier capteur et dudit deuxième capteur, un capteur de la position angulaire du support en mouvement, lesdits capteurs de mesures étant des capteurs inertiels (21c, 22c, 23c) distribués le long du support (2) entre la source de rayonnement (3) et le détecteur (4), les deux premier et deuxième capteurs étant fixés à chaque extrémité (2₁, 2₂) du support en mouvement (2) et le capteur de la position angulaire étant fixés à mi-chemin entre les deux extrémités (2₁, 2₂),
• Un module de synchronisation (23) adapté à déclencher de manière synchrone les mesures des capteurs,
• Un module de prétraitement (25) des mesures des capteurs, ledit module de prétraitement comprenant une entrée (25e) recevant un modèle M de fonctionnement du dispositif et un algorithme de fusion des données (27) prenant en compte au moins les mesures des capteurs et le modèle M pour estimer une valeur de position précise pour la source Ps, et pour le détecteur Pd, l'algorithme de fusion des données (27) étant configuré pour calibrer ledit dispositif D en ligne, en temps réel, sans requérir à des calibrations hors ligne a priori.

2. - Système selon la revendication 1 **caractérisé en ce que** le support en mouvement (2) est un bras et **en ce qu'**il comporte un encodeur (20) adapté à mesurer une position Pa angulaire du mouvement du bras.

3. - Système selon la revendication 1 **caractérisé en ce que** le support en mouvement (2) est un bras et **en ce qu'**il comporte N encodeurs (20), avec N supérieur ou égal à 1, le nombre N étant égal au nombre de degrés de liberté du système.

4. - Système selon l'une des revendications précédentes **caractérisé en ce que** l'algorithme de fusion des données est un algorithme de Kalman ou de Kalman étendu.

5. - Système selon l'une des revendications précédentes **caractérisé en ce que** le support en mouvement (2) est un bras en forme de C.

6. - Procédé pour calibrer en ligne un dispositif médical à rayons X (D) selon l'une des revendications 1 à 5, le procédé comprenant au moins les étapes suivantes :
• Enregistrer simultanément des mesures effectuées par au moins un premier capteur inertiel proche de la source de rayonnement, un deuxième capteur inertiel proche du détecteur et un capteur inertiel adapté à mesurer la position angulaire du support en mouvement, lesdits capteurs étant distribués le long du support (2) entre la source de rayonnement (3) et le détecteur (4), les deux premier et deuxième capteurs étant positionnés à chaque extrémité (2₁, 2₂) du support en mouvement (2) et le capteur de la position angulaire (23c) à mi-chemin entre les deux extrémités (2₁, 2₂),
• Transmettre ces mesures, ainsi qu'un modèle M de fonctionnement du dispositif D à un module configuré pour fusionner des données des mesures synchrones dudit premier capteur et dudit deuxième capteur afin d'estimer en ligne, en temps réel, une valeur de position Pd pour le détecteur et Ps la source ainsi qu'une erreur de mesure, sans requérir à des calibrations hors ligne a priori.

7. - Procédé selon la revendication 6 **caractérisé en ce que** l'on utilise un filtre de Kalman ou de Kalman étendu pour fusionner l'ensemble des données.

8. - Procédé selon l'une des revendications 6 ou 7 **caractérisé en ce que** l'on utilise des capteurs inertiels pour effectuer des mesures.

9. - Procédé selon l'une des revendications 6 ou 7 **caractérisé en ce que** l'on fusionne les mesures données par les capteurs proches de la source et de l'extrémité, avec la mesure de N encodeurs (20), avec N supérieur ou égal à 1, le nombre N étant égal au nombre de degrés de liberté du système.

10. - Procédé selon l'une des revendications 6 à 9 **caractérisé en ce que** l'on utilise un capteur optoélectronique pour mesurer en plus des déformations mécaniques du support en mouvement.

11. - Procédé selon la revendication 6 **caractérisé en ce que** l'on utilise un ou plusieurs réseaux de Bragg ou interféromètres pour déterminer une déformation du support en mouvement.

12. - Procédé selon l'une des revendications 6 à 9 **caractérisé en ce que** pour mesurer la déformation mécanique du support en mouvement (2) on utilise un dispositif choisi parmi la liste suivante : un ou plusieurs interféromètres et télémètres lasers, un ou plusieurs capteurs RFID/Ultrason.

## Patentansprüche

1. Kalibriersystem einer medizinischen Röntgenvorrichtung (D), umfassend mindestens einen beweglichen Träger (2), eine Strahlungsquelle (3) und einen Detektor (4), wobei der bewegliche Träger (2) zwei Enden (2₁, 2₂) umfasst, wobei die Strahlungsquelle (3) und der Detektor (4) an jedem Ende (2₁, 2₂) auf dem mindestens einen beweglichen Träger (2) montiert sind, wobei der bewegliche Träger (2) über mindestens eine Drehachse mit einer Basis (1) verbunden ist, **dadurch gekennzeichnet, dass** es mindestens die folgenden Elemente umfasst:
• Mindestens einen ersten Sensor (21c), der in der Nähe der Strahlungsquelle (3) angeordnet ist, und mindestens einen zweiten Sensor (22c), der in der Nähe des Detektors (4) angeordnet ist, wobei der erste und der zweite Sensor dafür geeignet sind, eine Position Ps der Quelle und eine Position Pd des Detektors durch Berechnung zu schätzen, indem Messungen von dem ersten Sensor und dem zweiten Sensor zusammengeführt werden, einen Winkelpositionssensor des beweglichen Trägers, wobei die Messungssensoren Trägheitssensoren (21c, 22c, 23c) sind, die entlang des Trägers (2) zwischen der Strahlungsquelle (3) und dem Detektor (4) verteilt sind, wobei der erste und der zweite Sensor an jedem Ende (2₁, 2₂) des beweglichen Trägers (2) befestigt sind und der Winkelpositionssensor auf halbem Weg zwischen den beiden Enden (2₁, 2₂) befestigt ist,
• Ein Synchronisationsmodul (23), das dafür geeignet ist, die Messungen der Sensoren synchron auszulösen,
• Ein Vorbehandlungsmodul (25) der Messungen der Sensoren, wobei das Vorbehandlungsmodul einen Eingang (25e) umfasst, der ein Funktionsmodell M der Vorrichtung und einen Datenzusammenführungsalgorithmus (27) unter Berücksichtigung mindestens der Messungen der Sensoren und des Modells M umfasst, um einen genauen Positionswert für die Quelle Ps und für den Detektor Pd zu schätzen, wobei der Datenzusammenführungsalgorithmus (27) dafür konfiguriert ist, die Vorrichtung D online in Echtzeit zu kalibrieren, ohne a priori Kalibrierungen offline zu benötigen.

2. System nach Anspruch 1, **dadurch gekennzeichnet, dass** der bewegliche Träger (2) ein Arm ist und dass es ein Codiergerät (20) aufweist, das dafür geeignet ist, eine Winkelposition Pa der Bewegung des Arms zu messen.

3. System nach Anspruch 1, **dadurch gekennzeichnet, dass** der bewegliche Träger (2) ein Arm ist und dass es N Codiergeräte (20) aufweist, wobei N größer oder gleich 1 ist, wobei die Zahl N gleich der Zahl der Freiheitsgrade des Systems ist.

4. System nach einem der vorhergehenden Ansprüche, **dadurch gekennzeichnet, dass** der Datenzusammenführungsalgorithmus ein Kalman-Algorithmus oder ein erweiterter Kalman-Algorithmus ist.

5. System nach einem der vorhergehenden Ansprüche, **dadurch gekennzeichnet, dass** der bewegliche Träger (2) ein Arm in C-Form ist.

6. Verfahren zum Online-Kalibrieren einer medizinischen Röntgenvorrichtung (D) nach einem der Ansprüche 1 bis 5, wobei das Verfahren mindestens die folgenden Schritte umfasst:
• Gleichzeitiges Speichern von Messungen, die von mindestens einem ersten Trägheitssensor nahe der Strahlungsquelle, einem zweiten Trägheitssensor nahe dem Detektor und einem Trägheitssensor, der dafür geeignet ist, die Winkelposition des beweglichen Trägers zu messen, durchgeführt werden, wobei die Sensoren entlang des Trägers (2) zwischen der Strahlungsquelle (3) und dem Detektor (4) verteilt sind, wobei der erste und der zweite Sensor an jedem Ende (2₁, 2₂) des beweglichen Trägers (2) und der Winkelpositionssensor (23c) auf halbem Weg zwischen den beiden Enden (2₁, 2₂) positioniert sind,
• Übertragen dieser Messungen sowie eines Funktionsmodells M der Vorrichtung D an ein Modul, das dafür konfiguriert ist, Daten der synchronen Messungen des ersten Sensors und des zweiten Sensors zusammenzuführen, um online in Echtzeit einen Positionswert Pd für den Detektor und Ps die Quelle sowie einen Messungsfehler zu schätzen, ohne a priori offline Kalibrierungen zu benötigen.

7. Verfahren nach Anspruch 6, **dadurch gekennzeichnet, dass** ein Kalman-Filter oder ein erweiterter Kalman-Filter zum Zusammenführen des Datensatzes verwendet wird.

8. Verfahren nach einem der Ansprüche 6 oder 7, **dadurch gekennzeichnet, dass** Trägheitsfilter zum Durchführen der Messungen verwendet werden.

9. Verfahren nach einem der Ansprüche 6 oder 7, **dadurch gekennzeichnet, dass** die von den Sensoren nahe der Quelle und des Endes gegebenen Messungen mit der Messung von N Codiergeräten (20) zusammengeführt werden, wobei N größer oder gleich 1 ist, wobei die Zahl N gleich der Zahl von Freiheitsgraden des Systems ist.

10. Verfahren nach einem der Ansprüche 6 bis 9, **dadurch gekennzeichnet, dass** ein optoelektronischer Sensor verwendet wird, um zusätzlich mechanische Verformungen des beweglichen Trägers zu messen.

11. Verfahren nach Anspruch 6, **dadurch gekennzeichnet, dass** ein oder mehrere Bragg-Gitter oder Interferometer verwendet werden, um eine Verformung des beweglichen Trägers zu bestimmen.

12. Verfahren nach einem der Ansprüche 6 bis 9, **dadurch gekennzeichnet, dass** zum Messen der mechanischen Verformung des beweglichen Trägers (2) eine Vorrichtung verwendet wird, die aus der folgenden Liste ausgewählt ist: ein oder mehrere Interferometer und Laser-Entfernungsmesser, ein oder mehrere RFID-/Ultraschall-Sensoren.

## Claims

1. A system for calibrating a medical X-ray device (D) comprising at least one moving support (2), one radiation source (3) and a detector (4), said moving support (2) comprising two ends (2₁, 2₂), the radiation source (3) and the detector (4) being mounted on said at least one moving support (2) at each end (2₁, 2₂), the moving support (2) being linked to a base (1) via at least one axis of rotation **characterised in that** it comprises at least the following elements:
• At least one first sensor (21c) arranged close to the radiation source (3) and at least one second sensor (22c) arranged close to the detector (4), the first and second sensors being suitable to estimate by calculation a position Ps of the source and a position Pd of the detector by merging measurements from said first sensor and said second sensor, a sensor of the angular position of the moving support, said measurement sensors being inertial sensors (21c, 22c, 23c) distributed along the support (2) between the radiation source (3) and the detector (4), the two first and second sensors being fixed to each end (2₁, 2₂) of the moving support (2) and the sensor of the angular position being fixed halfway between the two ends (2₁, 2₂),
• A synchronisation module (23) suitable to synchronously trigger the measurements of the sensors,
• A module for pre-processing (25) the measurements of the sensors, said pre-processing module comprising an input (25e) receiving an operating model M of the device and a data merging algorithm (27) taking into account at least the measurements of the sensors and the model M in order to estimate an accurate position value for the source Ps and for the detector Pd, the data merging algorithm (27) being configured to calibrate said device D online, in real time, without requiring offline calibrations a priori.

2. The system according to claim 1, **characterised in that** the moving support (2) is an arm and **in that** it has an encoder (20) suitable to measure an angular position Pa of the movement of the arm.

3. The system according to claim 1, **characterised in that** the moving support (2) is an arm and **in that** it has N encoders (20), with N being greater than or equal to 1, the number N being equal to the number of degrees of freedom of the system.

4. The system according to one of the preceding claims, **characterised in that** the data merging algorithm is a Kalman algorithm or an extended Kalman algorithm.

5. The system according to one of the preceding claims, **characterised in that** the moving support (2) is a C-arm.

6. A method for online calibration of a medical X-ray device (D) according to one of claims 1 to 5, the method comprising at least the following steps:
• Simultaneously recording measurements performed by at least one first inertial sensor near the radiation source, a second inertial sensor near the detector and an inertial sensor suitable to measure the angular position of the moving support, said sensors being distributed along the support (2) between the radiation source (3) and the detector (4), the first and second sensors being positioned at each end (2₁, 2₂) of the moving support (2) and the angular position sensor (23c) halfway between the two ends (2₁, 2₂),
• Transmitting these measurements as well as an operating model M of the device D to a module configured to merge data of the synchronous measurements of said first sensor and said second sensor in order to estimate online, in real time, a position value Pd for the detector and Ps the source as well as a measurement error, without requiring offline calibrations a priori.

7. The method according to claim 6, **characterised in that** a Kalman filter or an extended Kalman filter is used to merge all of the data.

8. The method according to one of claims 6 or 7, **characterised in that** inertial sensors are used to perform measurements.

9. The method according to one of claims 6 or 7, **characterised in that** the measurements given by the sensors near the source and the end are merged with the measurement of N encoders (20), with N greater than or equal to 1, the number N being equal to the number of degrees of freedom of the system.

10. The method according to one of claims 6 to 9, **characterised in that** an optoelectronic sensor is used to additionally measure mechanical deformations of the moving support.

11. The method according to claim 6, **characterised in that** one or more Bragg gratings or interferometers are used to determine deformation of the moving support.

12. The method according to one of claims 6 to 9, **characterised in that**, to measure the mechanical deformation of the moving support (2), a device chosen from the following list is used: one or more interferometers and laser telemeters, one or more RFID/ultrasound sensors.
